# EUROPEAN PATENT SPECIFICATION

(11) **EP 3 222 278 B1**
(45) Date of publication and mention of the grant of the patent: **14.06.2023**
(21) Application number: 15852781.2
(22) Date of filing: 23.10.2015
(51) Int. Cl.: A61K 31/4422, A61P 35/00, A61P 35/04

(54) **USE OF AZELNIDIPINE IN PREPARING MEDICINAL COMPOSITION FOR TREATING CANCERS**
VERWENDUNG VON AZELNIDIPIN ZUR HERSTELLUNG EINER MEDIZINISCHEN ZUSAMMENSETZUNG ZUR BEHANDLUNG VON KREBS
UTILISATION DE L'AZELNIDIPINE POUR LA PRÉPARATION D'UNE COMPOSITION PHARMACEUTIQUE INDIQUÉE POUR LE TRAITEMENT DU CANCER

(30) Priority: 24.10.2014 US 201462068298 P
(43) Date of publication of application: 27.09.2017
(73) Proprietor: Launx Biomedical Co., Ltd., Kaohsiung City 801 (TW)
(72) Inventor: CHEN, Chiu-Hung, Kaohsiung City 801 (TW); CHUANG, Show-Mei, Taichung City 408 (TW); WAY, Tzong-Der, Kaohsiung City 801 (TW); HSIAO, Nai-Wan, Taichung City 402 (TW)
(74) Representative: Mewburn Ellis LLP
(86) International application number: PCT/CN2015/092714
(87) International publication number: WO 2016/062275

(56) References cited:
- EP-A1- 3 222 278
- WO-A1-2015/157471
- WO-A1-2016/062275
- CN-A- 101 690 816
- CN-A- 103 930 132
- HUANG, WENJING;: 'Effect of Amlodipine on Cell Cycle and Cyclin Expression of Human Breast Cancer MCF-7 Cells and Regulation Mechanism of the Effect' CHINESE JOURNAL OF BIOLOGICALS vol. 23, no. 3, 31 March 2010, XP009502395
- YUN JUNG CHOI ET AL: "Combined inhibition of IGFR enhances the effects of gefitinib in H1650: a lung cancer cell line with EGFR mutation and primary resistance to EGFR-TK inhibitors", CANCER CHEMOTHERAPY AND PHARMACOLOGY, SPRINGER, BERLIN, DE, vol. 66, no. 2, 18 November 2009 (2009-11-18), pages 381-388, XP019843316, ISSN: 1432-0843
- K Foster: "Characterization of the A549 Cell Line as a Type II Pulmonary Epithelial Cell Model for Drug Metabolism", Experimental Cell Research, vol. 243, no. 2, 15 September 1998 (1998-09-15), pages 359-366, XP055003192, ISSN: 0014-4827, DOI: 10.1006/excr.1998.4172

## Description

### FIELD

The present disclosure related to a new indication of Azelnidipine pharmaceutical composition, especially related to inhibition effect of Azelnidipine pharmaceutical composition on a variety of cancer cells.

### BACKGROUND

Azelnidipine is a drug for treating hypertension and can be used alone or in combination with other antihypertensive drugs. Azelnidipine is approved by FDA and accumulated a huge data of drug use and drug mechanism research.

Due to the differences of the clinical use, there is no research present that the Azelnidipine has any potential to inhibit cancer cell.

On the other side, cancer is the most popular disease cause of death in the world. The cancer patients are gradually increase yearly, therefore the treatment method of the cancer has become an important issue. The medical treatments of cancer can be classified as surgical treatment, radiation therapy, chemotherapy and target therapy. Generally, the cancer drug, whether chemotherapy drug or target therapy drug, is to inhibit cancer cells duplication and split to prevent the tumor growth and metastasis. Averagely, only about five of 10,000 new drugs can successfully enter the phase I of clinical trials. Furthermore, if the cancer patients happen the drug resistance, that would reduce the effectiveness of the drugs and result in the medical treatment failure. Therefore, the new drug development is very difficult.

Therefore, it is a very urgent and important issue that how to develop anti-cancer drugs quickly and reduce the probability of clinical failure for treating various cancers.

WO 2015/157471 A1 relates to "iNOS-inhibitory compositions and their use as breast cancer therapeutics".

CN 103930132 A relates to "methods for treating or ameliorating the effects of a glioma"

### SUMMARY

The present invention is as defined in the claims. This invention and other aspects of the present disclosure may be more fully understood by reference to the following description.

In order to solve the above problems, the present disclosure provide the development of new cancer clinical indications of Azelnidipine.

Accordingly, the present disclosure provides a new indication of Azelnidipine. The experimental results showed that the Azelnidipine had no toxicity or had little toxicity to normal cells in the present disclosure. However, the selective effect of Azelnidipine between normal cells and cancer cells need to be identified.

The present disclosure provides a pharmaceutical composition of Azelnidipine for treating cancer. The pharmaceutical composition is composed of effective dose of Azelnidipine and a pharmaceutical acceptable salt.

In one embodiment of the present disclosure, the cancer is selected from pleural-related cancer, abdominal-related cancer, endocrine-related cancer, gastrointestinal tract-related cancer.

In one embodiment of the present disclosure, the cancer is selected from osteosarcoma, skin cancer and blood cancer.

In one embodiment of the present disclosure, the pleural-related cancer is lung cancer.

In one embodiment of the present disclosure, the abdominal-related cancer is selected from bladder cancer, cervical cancer, and kidney cancer.

In one embodiment of the present disclosure, the endocrine-related cancer is selected from prostate cancer, breast cancer, and ovarian cancer.

In one embodiment of the present disclosure, the gastrointestinal tract-related cancer is selected from gastric cancer, hepatic cancer, colorectal cancer, pancreatic cancer, and tongue cancer.

In one embodiment of the present disclosure, the effective dose of Azelnidipine is from 20 mg/kg/day to 500 mg/kg/day.

### BRIEF DESCRIPTION OF THE DRAWINGS

FIG 1 shows the results of the inhibitory effect of the different cancer cells by Azelnidipine.
FIG 2 shows the results of the inhibitory effect of tumor volume by Azelnidipine.
FIG 3 shows the inhibitory effect of tumor growth via administered high-dose and low-dose of Azelnidipine.

### DETAILED DESCRIPTION

### Cell culture

Subculture the different types of cancer cell lines. The cancer cells includes lung cancer, gastric cancer, hepatic cancer, colon cancer, skin cancer, cervical cancer, prostate cancer, bladder cancer, breast cancer, leukemia, pancreatic cancer, ovarian cancer, tongue cancer, osteosarcoma, and renal cancer. The normal cells used in the control group included kidney cell (HEK293) and human bronchial epithelial cell line BEAS-2B (as shown in Table 1).

Cancer cell lines were cultured in different culture medium according to different characteristics (as shown in Table 1). The cell numbers were counted and reseed as 2×10⁶in cell culture plate/flask. Then, the culture medium were added to a volume of 10 ml, and the cells were cultured for 2-3 days. Then, the cells were suspended for loading into 96-well plates. The number of cells was 3000 and the volume of the culture medium was 100µl each well.

**Table 1. Cancer cell lines and the culture medium**

| No | Cancer type | Cancer cell type | Culture medium |
|---|---|---|---|
| 1 | lung cancer | H1650 (lung adenocarcinoma) | RPMI-1640 |
| | | A549 (lung adenocarcinoma) | DMEM |
| 2 | gastric cancer | AGS (Gastric Adenocarcinoma ) | RPMI-1640 |
| | | MKN-45 (Gastric Adenocarcinoma) | RPMI-1640 |
| 3 | hepatic cancer | HepG2 (hepatocellular carcinoma) | DMEM |
| | | Hep3B (hepatocellular carcinoma) | DMEM |
| 4 | colon cancer | HCT116(p53+) (colorectal carcinoma) | DMEM |
| | | LoVo(Colorectal Adenocarcinoma) | DMEM |
| 5 | skin cancer | A375 ( amelanotic melanoma) | DMEM |
| | | BCC (basal cell carcinoma) | DMEM |
| 6 | cervical cancer | HeLa (Cervix Adenocarcinoma) | DMEM |
| | | C-33A (Cervical carcinoma) BCRC60554 | MEM |
| 7 | prostate cancer | PC3 (p53-)(Prostate adenocarcinoma) | DMEM |
| | | LNCaP clone FGC (LNCap.FGC) | RPMI-1640 |
| 8 | bladder cancer | 8301 (urinary bladder carcinoma) | RPMI-1640 |
| | | T24 | RPMI-1640 |
| 9 | breast cancer | MCF7 (Mammary Gland, Adenocarcinoma) | DMEM |
| | | MDA-MB-231 (Mammary Gland, Adenocarcinoma) | DMEM |
| 10 | pancreatic cancer | BxPC-3 | RPMI-1640 |
| | | AsPC-1 | RPMI-1640 |
| 11 | ovarian cancer | NIH:OVCAR-3 | RPMI-1640 |
| | | TOV-21G | RPMI-1640 |
| 12 | tongue cancer | SAS (Tongue squamous cell carcinoma) | DMEM |
| 13 | osteosarcoma | U-2OS | DMEM |
| 14 | renal cancer | 786-O (Renal adenocarcinoma) BCRC 60243 | RPMI-1640 |
| 15 | normal cell kidney pulmonary epithelial cell line | HEK293 (Kidney) | DMEM |
| | | BEAS-2B (Lung Epithelial) | RPMI-1640 |

### Cell viability analysis

Removing the original culture medium from 96-well plate. Then add 100 µl of commercially drug at a concentration of 10 µM per well. After 72 hours, add the diluted WST-1 reagent to the well with 100 µl/well, and the diluted WST-1 reagent was acquired from the dilution of 9:1 medium and WST-1 stock reagent. Finally, the total volume of each well was 200 µl/well. Culture the 96-well plate at 37 °C for 30 to 90 minutes. Detecting and calculate the survival rate of each cancer cells with an ELISA reader at OD450 nm.

### The effect of Azelnidipine on different cancer cell lines

### The inhibition effect of Azelnidipine on pleural-related cancer cells

This inhibition test of Azelnidipine on pleural-related cancer cells were using two lung cancer cell lines A549 and H1650. The inhibitory tests of Azelnidipine were performed 4 times for each cell lines and then the average value of the inhibitory tests was calculated. The results were shown in Table 2.

**Table 2. The inhibition effect of Azelnidipine on pleural-related cancer cells**

| | | | | | |
|---|---|---|---|---|---|
| | 0524-10 min | 0526-10 min | 0529-10 min | 0531-10 min | Average |
| A549 | 81.4 | 118.6 | 69.7 | 80.2 | 87.5 |
| | 1-10 min | 2-20 min | 3-20 min | 4-20 min | Average |
| H1650 | 28.8 | 26.5 | 40.5 | 62.7 | 39.7 |

### The inhibition effect of Azelnidipine on abdominal-related cancer cell lines

This inhibition test of Azelnidipine on abdominal-related cancer cells were using bladder cancer cell line TSGH and T24 (Table 3), cervical cancer cell lines HeLa and C-33A (Table 4), renal cancer cell line 786-O (Table 5). The inhibitory tests of Azelnidipine were performed 4 times for each cell lines and then the average value of the inhibitory tests was calculated. The results were shown in Table 3, Table 4, and Table 5.

**Table 3. The inhibition effect of Azelnidipine on bladder cancer cell lines**

| | | | | | |
|---|---|---|---|---|---|
| | 0510-10 min | 0512-10 min | 0515-10 min | 0517-10 min | average |
| TSGH | 151.0 | 117.3 | 72.0 | 120.1 | 115.1 |
| | 1-30 min | 2-20 min | 3-20 min | 4-20 min | average viability |
| T24 | 109.7 | 75.3 | 30.4 | 102.5 | 79.5 |

**Table 4. The inhibition effect of Azelnidipine on cervical cancer cell lines**

| | | | | | |
|---|---|---|---|---|---|
| | 0524-10 min | 0526-10 min | 0529-10 min | 0531-10 min | Average |
| HeLa | 70.4 | 93.7 | 81.4 | 102.7 | 87.0 |
| C-33A | 141.8 | 110.6 | 123.4 | 117.4 | 123.3 |

**Table 5. The inhibition effect of Azelnidipine on renal cancer cell line**

| | | | | | |
|---|---|---|---|---|---|
| | 0524-10 min | 0526-10 min | 0529-10 min | 0531-10 min | average |
| 786-O | 88.6 | 79.7 | 66.5 | 112.5 | 86.8 |

### The inhibition effect of Azelnidipine on endocrine-related cancer cell lines

This inhibition test of Azelnidipine on endocrine-related cancer cells were using prostate cancer cell line PC-3 (Table 6), breast cancer cell lines MCF7 and MDA-MB-231 (Table 7), and ovarian cancer cell lines NIH-OVCAR-3 and TOV-21G (Table 8). The inhibitory tests of Azelnidipine were performed 4 times for each cell lines and then the average value of the inhibitory tests was calculated. The results were shown in Table 6, Table 7, and Table 8.

**Table 6. The inhibition effect of Azelnidipine on prostate cancer cell line**

| | PC-3-0524-10 min | PC-3-0526-10 min | PC-3-0529-10 min | PC-3-0531-10 min | Average |
|---|---|---|---|---|---|
| PC-3 | 122.1 | 149.4 | 110.2 | 102.0 | 120.9 |

**Table 7. The inhibition effect of Azelnidipine on breast cancer cell lines**

| | | | | | |
|---|---|---|---|---|---|
| | 0612-10 min | 0614-10 min | 0616-10 min | 0619-10 min | average |
| MCF7 | 48.6 | 46.6 | 68.2 | 79.9 | 60.8 |
| | 0612-10 min | 0614-10 min | 0616-10 min | | average |
| MDA-MB-231 | 70.3 | 89.7 | 100.8 | | 86.9 |

**Table 8. The inhibition effect of Azelnidipine on ovarian cancer cell lines**

| | | | | | |
|---|---|---|---|---|---|
| | 7-3-30 min | 7-4-30 min | 7-7-30 min | 7-4-30 min | average |
| NIH-OVCAR-3 | 106.3 | 99.1 | 100.9 | 103.5 | 102.4 |
| | 7-3-30 min | 7-4-30 min | 7-7-30 min | 4-30 min | average |
| TOV-21G | 15.6 | 13.3 | 19.6 | 60.1 | 27.2 |

### The inhibition effect of Azelnidipine on gastrointestinal tract-related cancer cell lines

This inhibition test of Azelnidipine on gastrointestinal tract-related cancer cells were using gastric cancer cell lines AGS and MKN-45 (Table 9), hepatic cancer cell lines HepG2 and Hep3B (Table 10), colorectal cancer cell lines HCT116-wt and LoVo (Table 11), pancreatic cancer cell lines AsPC-1 and BxPC-3 (Table 12), tongue cancer cell line SAS (Table 13). The inhibitory tests of Azelnidipine were performed 4 times for each cell lines and then the average value of the inhibitory tests was calculated. The results were shown in Table 9, Table 10, Table 11, Table 12 and Table 13.

**Table 9. The inhibition effect of Azelnidipine on gastric cancer cell lines.**

| | | | | | |
|---|---|---|---|---|---|
| | 0510-10 min | 0512-10 min | 0515-10 min | 0517-10 min | average |
| AGS | 120.2 | 124.3 | 42.1 | 74.3 | 90.2 |
| | 0510-10 min | 0512-10 min | 0515-10 min | 0517-10 min | Average |
| MKN-45 | 173.5 | 183.7 | 68.5 | 130.3 | 139.0 |

**Table 10. The inhibition effect of Azelnidipine on hepatic cancer cell lines**

| | | | | | |
|---|---|---|---|---|---|
| | 0524-20 min | 0526-20 min | 0529-20 min | 0531-20 min | Average |
| HepG2 | 110.3 | 87.2 | 112.1 | 93.7 | 100.8 |
| | 0612-20 min | 0614-20 min | 0616-20 min | 0619-20 min | Average |
| Hep3B | 114.2 | 91.5 | 97.9 | 90.7 | 98.6 |

**Table 9. The inhibition effect of Azelnidipine on colorectal cancer cell lines**

| | | | | | |
|---|---|---|---|---|---|
| | 0602-30 min | 0605-10 min | 0607-10 min | 0609-10 min | Average |
| HCT116-wt | 70.7 | 146.2 | 158.9 | 69.3 | 111.3 |
| | 0616-10 min | 0619-10 min | 0621-10 min | 0623-10 min | Average |
| LoVo | 49.6 | 67.2 | 62.3 | 60.3 | 59.9 |

**Table 12. The inhibition effect of Azelnidipine on pancreatic cancer cell lines**

| | | | | | |
|---|---|---|---|---|---|
| | 1-7-3-30 min | 1-7-4-30 min | 1-7-7-30 min | 1-4-30 min | Average |
| AsPC-1 | 103.9 | 46.1 | 13.7 | 58.6 | 55.6 |
| | 3-7-3-30 min | 3-7-4-30 min | 3-7-7-30 min | 3-4-30 min | Average |
| BxPC-3 | 79.9 | 51.0 | 50.0 | 77.5 | 64.6 |

**Table 13. The inhibition effect of Azelnidipine on tongue cancer cell line**

| | | | | | |
|---|---|---|---|---|---|
| | 6-26-10 min | 6-28-10 min | 6-30-10 min | 7-3-10 min | Average |
| SAS | 69.9 | 66.0 | 66.7 | 104.7 | 76.8 |

### The inhibition effect of Azelnidipine on other cancer cell lines

This inhibition test of Azelnidipine on other cancer cells were using osteosarcoma cell line U2OS (Table 14), skin cancer cell lines A375 and BCC (Table 15). The inhibitory tests of Azelnidipine were performed 3 to 4 times for each cell lines and then the average value of the inhibitory tests was calculated. The results were shown in Table 14 and Table 15.

**Table 14. The inhibition effect of Azelnidipine on osteosarcoma cancer cell line**

| | | | | | |
|---|---|---|---|---|---|
| | 6-26-10 min | 6-28-10 min | 6-30-10 min | 7-3-10 min | Average |
| U2OS | 53.3 | 78.3 | 84.8 | 61.2 | 69.4 |

**Table 15. The inhibition effect of Azelnidipine on skin cancer cell lines**

| | | | | | |
|---|---|---|---|---|---|
| | 0602-30 min | 0605-10 min | 0607-10 min | 0609-10 min | Average |
| A375 | 123.6 | 100.2 | 76.0 | 104.9 | 101.2 |
| | 0602-30 min | 0605-10 min | 0607-10 min | | Average |
| BCC | 77.7 | 127.2 | 95.7 | | 100.2 |

### The experiment design on Control group

### The inhibition effect of Azelnidipine on normal cells

This inhibition test of Azelnidipine on normal cells were using normal kidney cell line HEK293 (Table 16) and normal pulmonary epithelial cell lines BEAS-2B (Table 17). The inhibitory tests of Azelnidipine were performed 4 times for each cell lines and then the average value of the inhibitory tests was calculated. The results were shown in Table 16 and Table 17.

**Table 16. The inhibition effect of Azelnidipine on normal kidney cell line**

| | Average |
|---|---|
| HEK293 | 94.2 |

**Table 17. The inhibition effect of Azelnidipine on normal pulmonary epithelial cell line**

| | | | | | |
|---|---|---|---|---|---|
| | 0510-10 min | 0512-10 min | 0515-10 min | 0517-10 min | average |
| BEAS-2B | 61.3 | 61.8 | 101.6 | 85.4 | 77.5 |

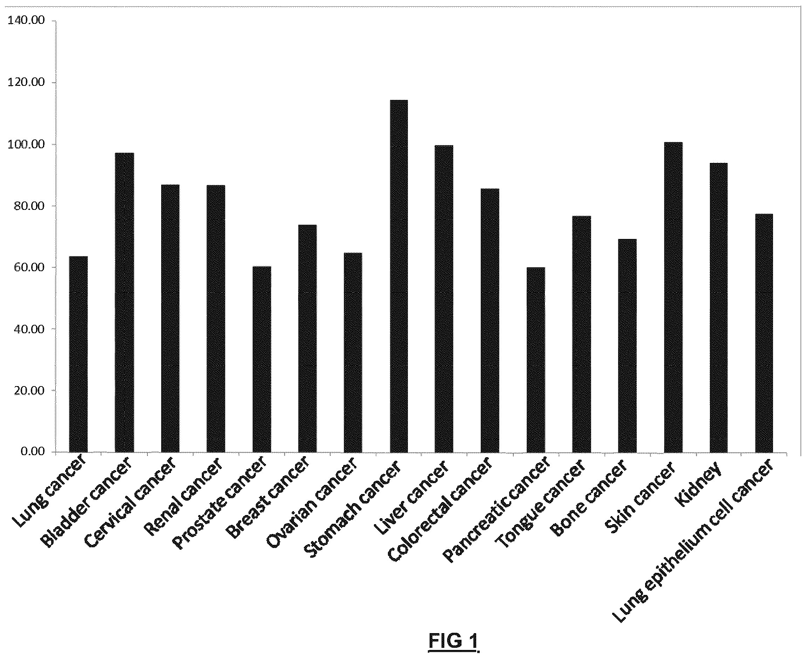

This inhibition test results of Azelnidipine on all kinds of cells were shown in Table 18. It is clear that Azelnidipine has a significant inhibitory effect on several cancers cell lines. As a result in the experiments of the present disclosure. Paroxetine has a significant inhibitory effect on various cancer cells (FIG 1).

**Table 18. Summary of the Effect on different cancer cell lines by Azelnidipine**

| | |
|---|---|
| cancer cells | Inhibitory effect |
| lung cancer | 63.60 |
| bladder cancer | 97.30 |
| cervical cancer | 87.00 |
| Kidney cancer | 86.80 |
| prostate cancer | 60.45 |
| breast cancer | 73.85 |
| ovarian cancer | 64.80 |
| gastric cancer | 114.60 |
| hepatic cancer | 99.70 |
| colorectal cancer | 85.60 |
| pancreatic cancer | 60.10 |
| tongue cancer | 76.80 |
| osteosarcoma | 69.40 |
| skin cancer | 100.70 |
| Normal cell | Inhibitory effect |
| Kidney cell | 94.20 |
| pulmonary epithelial cell line | 77.50 |

### Animal model test of ovarian cancer with dose 100 mg/kg/day and 200 mg/kg/day

In this disclosure, the female mice were( BALB / cAnN.Cg-Foxn1^{nu} / CrlNarl) purchased from National Laboratory Animal Center (Taiwan)). The weight of the mice were 21 ± 1 g. These mice were subcutaneously injected with gastric cancer cells (AGS) and then put these mice into different cages at random. The drug test experiment was divided into three groups, include "normal control group", "low dose group (100 mg/kg/day)", and "high dose group (200 mg/kg/day)". These mice were then injected test drug intraperitoneally once daily until the tumor size reached 100 mm³. The tumor sizes and body weight were measured twice a week. The tumor sizes were measured and calculated by formula :(L × W²). L represents the tumor longest length. W represents the tumor shortest diameter. The experiment results were shown in Table 19

**Table 19. The inhibitory effect of tumor volume via administered Azelnidipine**

| First measurement | | | | | | | | | | | | | | | |
|---|---|---|---|---|---|---|---|---|---|---|---|---|---|---|---|
| control group | | | | | | **low dose (100 mg/kg/day)** | | | | | **high dose (200 mg/kg/day)** | | | | |
| | **weight (g)** | **longest length mm** | **width mm** | **volume mm3** | tumor volume growth **mm3** | **weight (g)** | **longest length mm** | **width mm** | **volume mm3** | tumor volume growth **mm3** | **weight (g)** | **longest length mm** | **width mm** | **volume mm3** | tumor volume growth **mm3** |
| A | 18.5 | 7 | 7 | 171.5 | 171.5 | 21 | 8 | 8 | 256 | 256 | 20 | 4 | 3 | 18 | 18 |
| B | 22 | 8 | 6 | 144 | 144 | 21 | 6 | 7 | 147 | 147 | 19.5 | 6 | 3 | 27 | 27 |
| C | 20.5 | 9 | 8 | 288 | 288 | 21 | 7 | 6 | 126 | 126 | 20 | 4 | 4 | 32 | 32 |
| average | 20.4 | 7.6 | 7 | 189.3 | 189.3 | | | | 176.3333 | 176.3333 | | | | 25.66667 | 25.66667 |

| Second measurement | | | | | | | | | | | | | | | |
|---|---|---|---|---|---|---|---|---|---|---|---|---|---|---|---|
| control group | | | | | | **low dose (100 mg/kg/day)** | | | | | **high dose (200 mg/kg/day)** | | | | |
| | | | | | | | | | | | | | | | |

| | **weight (g)** | **longest length mm** | **width mm** | **volume mm3** | tumor volume growth **mm3** | **weight (g)** | **longest length mm** | **width mm** | **volume mm3** | tumor volume growth **mm3** | **weight (g)** | **longest length mm** | **width mm** | **volume mm3** | tumor volume growth **mm3** |
|---|---|---|---|---|---|---|---|---|---|---|---|---|---|---|---|
| A | 22 | 7 | 6 | 126 | -45.5 | 20 | 7 | 6 | 126 | -130 | 19 | 7 | 5 | 87.5 | 69.5 |
| B | 20 | 8 | 7 | 196 | 52 | 20 | 6 | 6 | 108 | -39 | 20 | 6 | 5 | 75 | 48 |
| C | 20 | 9 | 7 | 220.5 | -67.5 | 19 | 5 | 5 | 62.5 | -63.5 | 19 | 7 | 5 | 87.5 | 55.5 |
| average | 20.6 | 8.4 | 6.8 | 198.5 | 9.2 | | | | 98.83333 | -77.5 | | | | 83.33333 | 57.66667 |

| Third measurement | | | | | | | | | | | | | | | |
|---|---|---|---|---|---|---|---|---|---|---|---|---|---|---|---|
| | | | | | | | | | | | | | | | |

| control group | | | | | | **low dose (100 mg/kg/day)** | | | | | **high dose (200 mg/kg/day)** | | | | |
|---|---|---|---|---|---|---|---|---|---|---|---|---|---|---|---|
| | **weight** | **longest** | **width** | **volume** | Tumor volume | **weight** | **longest** | **width** | **volume** | Tumor | **weight** | **longest** | **width** | **volume** | Tumor |
| | **(g)** | **length mm** | **mm** | **mm3** | growth **mm3** | **(g)** | **length mm** | **mm** | **mm3** | volume growth **mm3** | **(g)** | **length mm** | **mm** | **mm3** | volume growth **mm3** |
| A | 23 | 9 | 6 | 162 | 36 | 19.5 | 7 | 6 | 126 | 0 | 20.5 | 7 | 5 | 87.5 | 0 |
| B | 20 | 10 | 8 | 320 | 124 | 19 | 6 | 6 | 108 | 0 | 19 | 5 | 5 | 62.5 | -12.5 |
| C | 21 | 11 | 7 | 269.5 | 49 | 18.5 | 5 | 5 | 62.5 | 0 | 20 | 0 | 0 | 0 | -87.5 |
| average | 21.2 | 10 | 6.8 | 235.3 | 36.8 | | | | 98.83333 | 0 | | | | 50 | -33.3333 |

| Fourth measurement | | | | | | | | | | | | | | | |
|---|---|---|---|---|---|---|---|---|---|---|---|---|---|---|---|
| control group | | | | | | **low dose (100 mg/kg/day)** | | | | | **high dose (200 mg/kg/day)** | | | | |
| | **weight (g)** | **longest length mm** | **width mm** | **volume mm3** | Tumor volume growth **mm3** | **weight (g)** | **longest length mm** | **width mm** | **volume mm3** | **Tumor** volume growth **mm3** | **weight (g)** | **longest length mm** | **width mm** | **volume mm3** | Tumor volume growth **mm3** |
| A | 23 | 11 | 7 | 269.5 | 107.5 | 20 | 4 | 3 | 18 | -108 | 20 | 0 | 0 | 0 | -87.5 |
| B | 22 | 10 | 6 | 180 | -140 | 21 | 5 | 4 | 40 | -68 | 20 | 0 | 0 | 0 | -62.5 |
| C | 23 | 11 | 8 | 352 | 82.5 | 20 | 0 | 0 | 0 | -88 | 21 | 0 | 0 | 0 | 0 |
| average | 22.4 | | | 233.5 | -1.8 | | | | 19.33333 | -88 | | | | 0 | -50 |

| Fifth measurement | | | | | | | | | | | | | | | |
|---|---|---|---|---|---|---|---|---|---|---|---|---|---|---|---|
| control group | | | | | | **low dose (100 mg/kg/day)** | | | | | **high dose (200 mg/kg/day)** | | | | |
| | **weight (g)** | **longest length mm** | **width mm** | **volume mm3** | Tumor volume growth **mm3** | **weight (g)** | **longest length mm** | **width mm** | **volume mm3** | tumor volume growth | **weight (g)** | **longest length mm** | **width mm** | **volume mm3** | Tumor volume grouth **mm3** |
| A | 22 | 12 | 8 | 384 | 114.5 | 20 | 4 | 3 | 18 | 0 | 20 | 0 | 0 | 0 | 0 |
| B | 22 | 11 | 8 | 352 | 172 | 20 | 6 | 4 | 48 | 8 | 20 | 0 | 0 | 0 | 0 |
| C | 23 | 12 | 9 | 486 | 134 | 21 | 0 | 0 | 0 | 0 | 21 | 0 | 0 | 0 | 0 |
| average | 22.4 | | | 295.7 | 62.2 | | | | 22 | 2.666667 | | | | 0 | 0 |

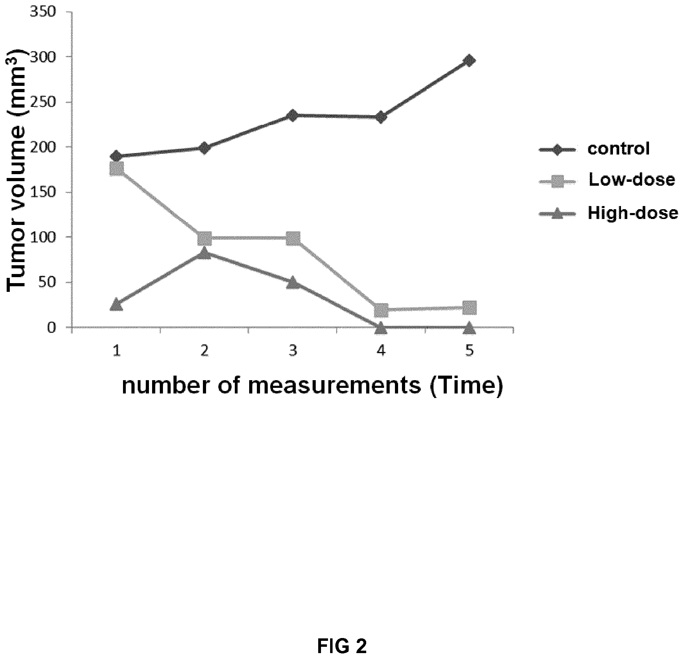

According to the results in FIG 2, low dose and high dose of Azelnidipine had significant inhibition effect on tumor cells. In the meantime, the weight of mice did not show a significant decrease during the experiment. These results indicated that both high and low doses of Azelnidipine could keep the tested mice in healthy status during the treatment without death.

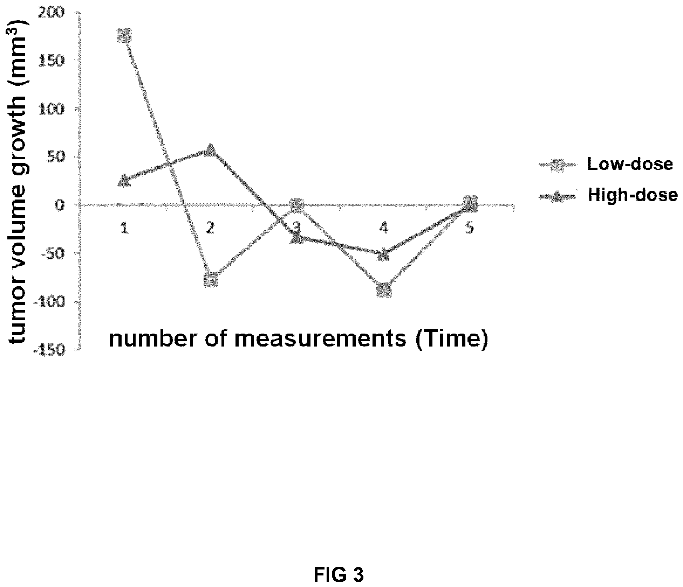

According to the results in FIG 3, low dose and high dose of Azelnidipine had effectively slow down the tumor volume growth, and can also reduce the tumor volume. Especially, high dose of Azelnidipine had better effect to inhibit tumor growth.

## Claims

1. A pharmaceutical composition for use in treating a cancer comprising a therapeutically effective amount of Azelnidipine or a pharmaceutical acceptable salt thereof, wherein the cancer is selected from the group consisting of ovarian cancer, pancreatic cancer, tongue cancer, osteosarcoma, and lung cancer.

## Patentansprüche

1. Pharmazeutische Zusammensetzung zur Verwendung bei der Behandlung von Krebs, die eine therapeutisch wirksame Menge von Azelnidipin oder einem pharmazeutisch annehmbaren Salz davon umfasst, wobei der Krebs aus der aus Ovarialkrebs, Pankreaskrebs, Zungenkrebs, Osteosarkomen und Lungenkrebs bestehenden Gruppe ausgewählt ist.

## Revendications

1. Composition pharmaceutique à utiliser dans le traitement d'un cancer comprenant une quantité thérapeutiquement efficace d'azelnidipine ou d'un sel pharmaceutiquement acceptable de celui-ci, dans laquelle le cancer est choisi dans le groupe comprenant le cancer de l'ovaire, le cancer du pancréas, le cancer de la langue, l'ostéosarcome et le cancer du poumon.
